Europäisches Patentamt

European Patent Office

Office européen des brevets

(19)

(11) **EP 1 152 011 A1**

(12) **EUROPEAN PATENT APPLICATION**
published in accordance with Art. 158(3) EPC

(43) Date of publication:
**07.11.2001 Bulletin 2001/45**

(51) Int Cl.⁷: **C07K 4/00**, C07K 14/47,
A61K 38/02, A61K 38/16

(21) Application number: **00902869.7**

(22) Date of filing: **09.02.2000**

(86) International application number:
**PCT/JP00/00703**

(87) International publication number:
**WO 00/47606 (17.08.2000 Gazette 2000/33)**

(84) Designated Contracting States:
**AT BE CH CY DE DK ES FI FR GB GR IE IT LI LU
MC NL PT SE**
Designated Extension States:
**AL LT LV MK RO SI**

(30) Priority: **12.02.1999 JP 3377299**

(71) Applicant: **KYOWA HAKKO KOGYO CO., LTD.
Chiyoda-ku, Tokyo 100-8185 (JP)**

(72) Inventors:
• **SHIBATA, Kenji, Kyowa Hakko Kogyo Co., Ltd.
Machida-shi, Tokyo 194-8533 (JP)**

• **YAMASAKI, Motoo,
Kyowa Hakko Kogyo Co., Ltd.
Machida-shi, Tokyo 194-8533 (JP)**
• **TSUKUDA, Eiji, Kyowa Hakko Kogyo Co., Ltd.
Nagaizumi-cho, Sunto-gun, Shizuoka 411-8 (JP)**
• **ODA, Shoji, Kyowa Hakko Kogyo Co., Ltd.
Chiyoda-ku, Tokyo 100-8185 (JP)**
• **MIYAMOTO, Kaoru
Maebashi-shi, Gunma 371-0822 (JP)**

(74) Representative: **VOSSIUS & PARTNER
Siebertstrasse 4
81675 München (DE)**

(54) **PEPTIDES INHIBITING VASCULAR ENDOTHELIAL CELL MIGRATION**

(57)    The present invention provides novel peptides inhibiting vascular endothelial cell migration, which are useful as an agent for treating diseases relating to pathological angiogenesis, for example, solid tumor, inflammatory diseases such as arthritis, ocular angiogenic diseases such as diabetic retinitis, or the like.

The present invention provides peptides having an activity of inhibiting vascular endothelial cell migration represented by the following formula (I):

$$R^1\text{-}A\text{-}R^2 \qquad (I)$$

wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a partial amino acid sequence of the amino acid sequence represented by any of SEQ ID NOs:1 to 3 wherein one or more amino acid residue may be substituted, deleted or added, or pharmaceutically acceptable salts thereof.

EP 1 152 011 A1

## SEQUENCE LISTING

<110> KYOWA HAKKO KOGYO CO., LTD.

<120> Vascular Endotherial Cell Migration Inhibitory Peptides

<130> 11186WO1

<140>

<141>

<160> 14

<170> PatentIn Ver. 2.0

<210> 1

<211> 807

<212> PRT

<213> Homo sapience

<400> 1

Met Arg Leu Ser Pro Ala Pro Leu Lys Leu Ser Arg Thr Pro Ala Leu
1               5                   10                  15

Leu Ala Leu Ala Leu Pro Leu Ala Ala Ala Leu Ala Phe Ser Asp Glu
            20                  25                  30

Thr Leu Asp Lys Val Pro Lys Ser Glu Gly Tyr Cys Ser Arg Ile Leu
35 40 45

Arg Ala Gln Gly Thr Arg Arg Glu Gly Tyr Thr Glu Phe Ser Leu Arg
50 55 60

Val Glu Gly Asp Pro Asp Phe Tyr Lys Pro Gly Thr Ser Tyr Arg Val
65 70 75 80

Thr Leu Ser Ala Ala Pro Pro Ser Tyr Phe Arg Gly Phe Thr Leu Ile
85 90 95

Ala Leu Arg Glu Asn Arg Glu Gly Asp Lys Glu Glu Asp His Ala Gly
100 105 110

Thr Phe Gln Ile Ile Asp Glu Glu Glu Thr Gln Phe Met Ser Asn Cys
115 120 125

Pro Val Ala Val Thr Glu Ser Thr Pro Arg Arg Arg Thr Arg Ile Gln
130 135 140

Val Phe Trp Ile Ala Pro Pro Ala Gly Thr Gly Cys Val Ile Leu Lys
145 150 155 160

Ala Ser Ile Val Gln Lys Arg Ile Ile Tyr Phe Gln Asp Glu Gly Ser
165 170 175

Leu Thr Lys Lys Leu Cys Glu Gln Asp Ser Thr Phe Asp Gly Val Thr
            180                 185                 190

Asp Lys Pro Ile Leu Asp Cys Cys Ala Cys Gly Thr Ala Lys Tyr Arg
            195                 200                 205

Leu Thr Phe Tyr Gly Asn Trp Ser Glu Lys Thr His Pro Lys Asp Tyr
        210                 215                 220

Pro Arg Arg Ala Asn His Trp Ser Ala Ile Ile Gly Gly Ser His Ser
225                 230                 235                 240

Lys Asn Tyr Val Leu Trp Glu Tyr Gly Gly Tyr Ala Ser Glu Gly Val
            245                 250                 255

Lys Gln Val Ala Glu Leu Gly Ser Pro Val Lys Met Glu Glu Glu Ile
            260                 265                 270

Arg Gln Gln Ser Asp Glu Val Leu Thr Val Ile Lys Ala Lys Ala Gln
        275                 280                 285

Trp Pro Ala Trp Gln Pro Leu Asn Val Arg Ala Ala Pro Ser Ala Glu
        290                 295                 300

Phe Ser Val Asp Arg Thr Arg His Leu Met Ser Phe Leu Thr Met Met
305                 310                 315                 320

Gly Pro Ser Pro Asp Trp Asn Val Gly Leu Ser Ala Glu Asp Leu Cys
                325                 330                 335

Thr Lys Glu Cys Gly Trp Val Gln Lys Val Val Gln Asp Leu Ile Pro
                340                 345                 350

Trp Asp Ala Gly Thr Asp Ser Gly Val Thr Tyr Glu Ser Pro Asn Lys
                355                 360                 365

Pro Thr Ile Pro Gln Glu Lys Ile Arg Pro Leu Thr Ser Leu Asp His
        370                 375                 380

Pro Gln Ser Pro Phe Tyr Asp Pro Glu Gly Gly Ser Ile Thr Gln Val
385                 390                 395                 400

Ala Arg Val Val Ile Glu Arg Ile Ala Arg Lys Gly Glu Gln Cys Asn
                405                 410                 415

Ile Val Pro Asp Asn Val Asp Asp Ile Val Ala Asp Leu Ala Pro Glu
                420                 425                 430

Glu Lys Asp Glu Asp Asp Thr Pro Glu Thr Cys Ile Tyr Ser Asn Trp
        435                 440                 445

Ser Pro Trp Ser Ala Cys Ser Ser Ser Thr Cys Asp Lys Gly Lys Arg
        450                 455                 460

Met Arg Gln Arg Met Leu Lys Ala Gln Leu Asp Leu Ser Val Pro Cys
465            470            475            480

Pro Asp Thr Gln Asp Phe Gln Pro Cys Met Gly Pro Gly Cys Ser Asp
                485            490            495

Glu Asp Gly Ser Thr Cys Thr Met Ser Glu Trp Ile Thr Trp Ser Pro
                500            505            510

Cys Ser Ile Ser Cys Gly Met Gly Met Arg Ser Arg Glu Arg Tyr Val
            515            520            525

Lys Gln Phe Pro Glu Asp Gly Ser Val Cys Thr Leu Pro Thr Glu Glu
            530            535            540

Met Glu Lys Cys Thr Val Asn Glu Glu Cys Ser Pro Ser Ser Cys Leu
545            550            555            560

Met Thr Glu Trp Gly Glu Trp Asp Glu Cys Ser Ala Thr Cys Gly Met
                565            570            575

Gly Met Lys Lys Arg His Arg Met Ile Lys Met Asn Pro Ala Asp Gly
                580            585            590

Ser Met Cys Lys Ala Glu Thr Ser Gln Ala Glu Lys Cys Met Met Pro
                595            600            605

Glu Cys His Thr Ile Pro Cys Leu Leu Ser Pro Trp Ser Glu Trp Ser
610                615                620

Asp Cys Ser Val Thr Cys Gly Lys Gly Met Arg Thr Arg Gln Arg Met
625                630                635                640

Leu Lys Ser Leu Ala Glu Leu Gly Asp Cys Asn Glu Asp Leu Glu Gln
645                650                655

Val Glu Lys Cys Met Leu Pro Glu Cys Pro Ile Asp Cys Glu Leu Thr
660                665                670

Glu Trp Ser Gln Trp Ser Glu Cys Asn Lys Ser Cys Gly Lys Gly His
675                680                685

Val Ile Arg Thr Arg Met Ile Gln Met Glu Pro Gln Phe Gly Gly Ala
690                695                700

Pro Cys Pro Glu Thr Val Gln Arg Lys Lys Cys Arg Ile Arg Lys Cys
705                710                715                720

Leu Arg Asn Pro Ser Ile Gln Lys Pro Arg Trp Arg Glu Ala Arg Glu
725                730                735

Ser Arg Arg Ser Glu Gln Leu Lys Glu Glu Ser Glu Gly Glu Gln Phe
740                745                750

Pro Gly Cys Arg Met Arg Pro Trp Thr Ala Trp Ser Glu Cys Thr Lys
        755                 760                 765

Leu Cys Gly Gly Gly Ile Gln Glu Arg Tyr Met Thr Val Lys Lys Arg
       770                 775                 780

Phe Lys Ser Ser Gln Phe Thr Ser Cys Lys Asp Lys Lys Glu Ile Arg
785                 790                 795                 800

Ala Cys Asn Val His Pro Cys
                805

<210> 2

<211> 807

<212> PRT

<213> Bovine


<400> 2

Met Arg Leu Ser Pro Val Leu Leu Arg Leu Ser Arg Gly Pro Ala Leu
    1                 5                 10                15

Leu Ala Leu Ala Leu Pro Leu Ala Val Ala Leu Ala Phe Ser Asp Glu
                20                25                30

Thr Leu Asp Lys Val Pro Lys Ser Glu Gly Tyr Cys Ser Arg Ile Leu
           35                 40                45

Arg Val Gln Gly Thr Arg Arg Glu Gly Tyr Thr Glu Phe Ser Leu Arg
    50                  55                  60

Val Glu Gly Asp Pro Asp Phe Tyr Lys Pro Gly Thr Ser Tyr Arg Val
 65                  70                  75                  80

Thr Leu Ser Ala Ala Pro Pro Ser Tyr Phe Arg Gly Phe Thr Leu Ile
                85                  90                  95

Ala Leu Lys Glu Asn Arg Glu Gly Asp Lys Glu Glu Asp His Ala Gly
                100                 105                 110

Thr Phe Gln Ile Ile Asp Glu Glu Glu Thr Gln Phe Met Ser Asn Cys
            115                 120                 125

Pro Val Ala Val Thr Glu Ser Thr Pro Arg Arg Arg Thr Arg Ile Gln
    130                 135                 140

Val Phe Trp Ile Ala Pro Pro Ala Gly Thr Gly Cys Val Ile Leu Lys
 145                 150                 155                 160

Ala Ser Ile Val Gln Lys Arg Ile Ile Tyr Phe Gln Asp Glu Gly Ser
                165                 170                 175

Leu Thr Lys Lys Leu Cys Glu Gln Asp Ser Thr Phe Asp Gly Val Thr
                180                 185                 190

Asp Lys Pro Ile Leu Asp Cys Cys Ala Cys Gly Thr Ala Lys Tyr Arg
        195                 200                 205

Leu Thr Phe Tyr Gly Asn Trp Ser Glu Lys Thr His Pro Lys Asp Tyr
        210                 215                 220

Pro Arg Arg Ala Asn His Trp Ser Ala Ile Ile Gly Gly Ser His Ser
225                 230                 235                 240

Lys Asn Tyr Val Leu Trp Glu Tyr Gly Gly Tyr Ala Ser Glu Gly Val
                245                 250                 255

Lys Gln Val Ala Glu Leu Gly Ser Pro Val Lys Met Glu Glu Glu Ile
                260                 265                 270

Arg Gln Gln Ser Asp Glu Val Leu Thr Val Ile Lys Ala Lys Ala Gln
        275                 280                 285

Trp Pro Ala Trp Gln Pro Leu Asn Val Arg Ala Ala Pro Ser Ala Glu
        290                 295                 300

Phe Ser Val Asp Arg Thr Arg His Leu Met Ser Phe Leu Thr Met Met
305                 310                 315                 320

Gly Pro Ser Pro Asp Trp Asn Val Gly Leu Ser Ala Glu Asp Leu Cys
                325                 330                 335

Thr Lys Glu Cys Gly Trp Val Gln Lys Val Val Gln Asp Leu Ile Pro
            340              345              350

Trp Asp Ala Gly Thr Asp Ser Gly Val Thr Tyr Glu Ser Pro Asn Lys
            355              360              365

Pro Thr Ile Pro Gln Glu Lys Ile Arg Pro Leu Thr Ser Leu Asp His
        370              375              380

Pro Gln Ser Pro Phe Tyr Asp Pro Glu Gly Gly Ser Ile Thr Gln Val
385              390              395              400

Ala Arg Val Val Ile Glu Arg Ile Ala Arg Lys Gly Glu Gln Cys Asn
                405              410              415

Ile Val Pro Asp Asn Val Asp Asp Ile Val Ala Asp Leu Ala Pro Glu
            420              425              430

Glu Lys Asp Glu Asp Asp Thr Pro Glu Thr Cys Ile Tyr Ser Asn Trp
        435              440              445

Ser Pro Trp Ser Ala Cys Ser Ser Ser Thr Cys Asp Lys Gly Lys Arg
        450              455              460

Met Arg Gln Arg Met Leu Lys Ala Gln Leu Asp Leu Ser Val Pro Cys
465              470              475              480

Pro Asp Thr Gln Asp Phe Gln Pro Cys Met Gly Pro Gly Cys Ser Asp
485 490 495

Glu Asp Gly Ser Thr Cys Thr Met Ser Glu Trp Ile Thr Trp Ser Pro
500 505 510

Cys Ser Ile Ser Cys Gly Thr Gly Thr Arg Ser Arg Glu Arg Tyr Val
515 520 525

Lys Gln Phe Pro Glu Asp Gly Ser Val Cys Thr Leu Pro Thr Glu Glu
530 535 540

Thr Glu Lys Cys Thr Val Asn Glu Glu Cys Ser Pro Ser Ser Cys Leu
545 550 555 560

Thr Thr Glu Trp Gly Glu Trp Asp Glu Cys Ser Ala Thr Cys Gly Met
565 570 575

Gly Met Lys Lys Arg His Arg Met Val Lys Met Ser Pro Ala Asp Gly
580 585 590

Ser Met Cys Lys Ala Glu Thr Ser Gln Ala Glu Lys Cys Met Met Pro
595 600 605

Glu Cys His Thr Ile Pro Cys Leu Leu Ser Leu Trp Ser Glu Trp Ser
610 615 620

Asp Cys Ser Val Thr Cys Gly Lys Gly Met Arg Thr Arg Gln Arg Met
625 630 635 640

Leu Lys Ser Leu Ala Glu Leu Gly Asp Cys Asn Glu Glu Leu Glu Gln
645 650 655

Val Glu Lys Cys Met Leu Pro Glu Cys Pro Ile Asp Cys Glu Leu Thr
660 665 670

Glu Trp Ser Gln Trp Ser Glu Cys Asn Lys Ser Cys Gly Lys Gly His
675 680 685

Met Ile Arg Thr Arg Met Ile Gln Met Glu Pro Gln Phe Gly Gly Thr
690 695 700

Pro Cys Pro Glu Thr Val Gln Arg Lys Lys Cys Arg Ile Arg Lys Cys
705 710 715 720

Leu Arg Asn Pro Ser Ile Gln Asn Leu Arg Trp Arg Glu Ala Arg Glu
725 730 735

Ser Arg Arg Ser Glu Gln Leu Arg Glu Glu Ser Asp Gly Asp Gln Phe
740 745 750

Pro Gly Cys Arg Met Arg Pro Trp Thr Ala Trp Ser Glu Cys Thr Lys
755 760 765

Leu Cys Gly Gly Gly Ile Gln Glu Arg Tyr Met Thr Val Lys Lys Arg
770              775              780

Phe Lys Ser Ser Gln Phe Thr Ser Cys Lys Asp Lys Lys Glu Ile Arg
785              790              795              800

Ala Cys Asn Val His Pro Cys
                805

<210> 3

<211> 807

<212> PRT

<213> Rat

<400> 3

Met Arg Leu Ser Pro Ala Pro Leu Arg Leu Ser Arg Gly Pro Ala Leu
1              5              10              15

Leu Ala Leu Ala Leu Pro Leu Ala Ala Ala Leu Ala Phe Ser Asp Glu
                20              25              30

Thr Leu Asp Lys Val Ala Lys Ser Glu Gly Tyr Cys Ser Arg Ile Leu
                35              40              45

Arg Ala Gln Gly Thr Arg Arg Glu Gly Tyr Thr Glu Phe Ser Leu Arg

50         55         60

Val Glu Gly Asp Pro Asp Phe Tyr Lys Pro Gly Ser Ser Tyr Arg Val

65         70         75         80

Thr Leu Ser Ala Ala Pro Pro Ser Tyr Phe Arg Gly Phe Thr Leu Ile

85         90         95

Ala Leu Lys Glu Asn Arg Glu Gly Asp Lys Glu Glu Asp His Ala Gly

100         105         110

Thr Phe Gln Ile Ile Asp Glu Glu Glu Thr Gln Phe Met Ser Asn Cys

115         120         125

Pro Val Ala Val Thr Glu Ser Thr Pro Arg Arg Arg Thr Arg Ile Gln

130         135         140

Val Phe Trp Ile Ala Pro Pro Thr Gly Thr Gly Cys Val Ile Leu Lys

145         150         155         160

Ala Ser Ile Val Gln Lys Arg Ile Ile Tyr Phe Gln Asp Glu Gly Ser

165         170         175

Leu Thr Lys Lys Leu Cys Glu Gln Asp Pro Thr Leu Asp Gly Val Thr

180         185         190

Asp Arg Pro Ile Leu Asp Cys Cys Ala Cys Gly Thr Ala Lys Tyr Arg

195                    200                    205

Leu Thr Phe Tyr Gly Asn Trp Ser Glu Lys Thr His Pro Lys Asp Tyr
     210                    215                    220

Pro Arg Arg Ala Asn His Trp Ser Ala Ile Ile Gly Gly Ser His Ser
225                    230                    235                    240

Lys Asn Tyr Val Leu Trp Glu Tyr Gly Gly Tyr Ala Ser Glu Gly Val
                    245                    250                    255

Lys Gln Val Ala Glu Leu Gly Ser Pro Val Lys Met Glu Glu Glu Ile
               260                    265                    270

Arg Gln Gln Ser Asp Glu Val Leu Thr Val Ile Lys Ala Lys Ala Gln
          275                    280                    285

Trp Pro Ser Trp Gln Pro Val Asn Val Arg Ala Ala Pro Ser Ala Glu
     290                    295                    300

Phe Ser Val Asp Arg Thr Arg His Leu Met Ser Phe Leu Thr Met Met
305                    310                    315                    320

Gly Pro Ser Pro Asp Trp Asn Val Gly Leu Ser Ala Glu Asp Leu Cys
                    325                    330                    335

Thr Lys Glu Cys Gly Trp Val Gln Lys Val Val Gln Asp Leu Ile Pro

                340                    345                    350

Trp Asp Ala Gly Thr Asp Ser Gly Val Thr Tyr Glu Ser Pro Asn Lys
        355                    360                    365

Pro Thr Ile Pro Gln Glu Lys Ile Arg Pro Leu Thr Ser Leu Asp His
        370                    375                    380

Pro Gln Ser Pro Phe Tyr Asp Pro Glu Gly Gly Ser Ile Thr Gln Val
385                    390                    395                    400

Ala Arg Val Val Ile Glu Arg Ile Ala Arg Lys Gly Glu Gln Cys Asn
                405                    410                    415

Ile Val Pro Asp Asn Val Asp Asp Ile Val Ala Asp Leu Ala Pro Glu
        420                    425                    430

Glu Lys Asp Glu Asp Asp Thr Pro Glu Thr Cys Ile Tyr Ser Asn Trp
        435                    440                    445

Ser Pro Trp Ser Ala Cys Ser Ser Ser Thr Cys Glu Lys Gly Lys Arg
        450                    455                    460

Met Arg Gln Arg Met Leu Lys Ala Gln Leu Asp Leu Ser Val Pro Cys
465                    470                    475                    480

Pro Asp Thr Gln Asp Phe Gln Pro Cys Met Gly Pro Gly Cys Ser Asp

485                     490                     495

Glu Asp Gly Ser Thr Cys Thr Met Ser Glu Trp Ile Thr Trp Ser Pro
        500                     505                     510

Cys Ser Val Ser Cys Gly Met Gly Met Arg Ser Arg Glu Arg Tyr Val
        515                     520                     525

Lys Gln Phe Pro Glu Asp Gly Ser Val Cys Met Leu Pro Thr Glu Glu
        530                     535                     540

Thr Glu Lys Cys Thr Val Asn Glu Glu Cys Ser Pro Ser Ser Cys Leu
545                     550                     555                     560

Val Thr Glu Trp Gly Glu Trp Asp Asp Cys Ser Ala Thr Cys Gly Met
            565                     570                     575

Gly Met Lys Lys Arg His Arg Met Val Lys Met Ser Pro Ala Asp Gly
            580                     585                     590

Ser Met Cys Lys Ala Glu Thr Ser Gln Ala Glu Lys Cys Met Met Pro
            595                     600                     605

Glu Cys His Thr Ile Pro Cys Leu Leu Ser Pro Trp Ser Glu Trp Ser
            610                     615                     620

Asp Cys Ser Val Thr Cys Gly Lys Gly Met Arg Thr Arg Gln Arg Met

625               630               635               640

Leu Lys Ser Leu Ala Glu Leu Gly Asp Cys Asn Glu Asp Leu Glu Gln

                 645               650              655

Ala Glu Lys Cys Met Leu Pro Glu Cys Pro Ile Asp Cys Glu Leu Ser

           660               665             670

Glu Trp Ser Gln Trp Ser Glu Cys Asn Lys Ser Cys Gly Lys Gly His

           675               680             685

Met Ile Arg Thr Arg Thr Ile Gln Met Glu Pro Gln Phe Gly Gly Ala

      690               695             700

Pro Cys Pro Glu Thr Val Gln Arg Lys Lys Cys Arg Ala Arg Lys Cys

705               710             715             720

Leu Arg Ser Pro Ser Ile Gln Lys Leu Arg Trp Arg Glu Ala Arg Glu

           725               730             735

Ser Arg Arg Ser Glu Gln Leu Arg Glu Glu Ser Asp Gly Glu Gln Phe

           740               745             750

Pro Gly Cys Arg Met Arg Pro Trp Thr Ala Trp Ser Glu Cys Thr Lys

           755               760             765

Leu Cys Gly Gly Gly Ile Gln Glu Arg Tyr Met Thr Val Lys Lys Arg

770     775     780

Phe Lys Ser Ser Gln Phe Thr Ser Cys Lys Asp Lys Lys Glu Ile Arg

785     790     795     800

Ala Cys Asn Val His Pro Cys

805

<210> 4

<211> 15

<212> PRT

<213> Artificial Sequence

<220>

<221> MOD_RES

<222> (15)

<223> AMIDATION,Glycine amide

<220>

<223> Synthetic Peptide

<400> 4

Met Ser Glu Trp Ile Thr Trp Ser Pro Ala Ser Ile Ser Ala Xaa

1     5     10     15

<210> 5

<211> 15

<212> PRT

<213> Artificial Sequence


<220>

<221> MOD_RES

<222> (15)

<223> AMIDATION,Glycine amide


<220>

<223> Synthetic Peptide


<400> 5

Leu Ser Leu Trp Ser Glu Trp Ser Asp Ala Ser Val Thr Ala Xaa
1              5               10              15


<210> 6

<211> 16

<212> PRT

<213> Artificial Sequence


<220>

<221> MOD_RES

<222> (16)

<223> AMIDATION,Aspartate amide

<220>

<223> Synthetic Peptide

<400> 6

Tyr Ser Asn Trp Ser Pro Trp Ser Ala Ala Ser Ser Ser Thr Ala Xaa
1               5                   10                  15

<210> 7

<211> 15

<212> PRT

<213> Artificial Sequence

<220>

<221> MOD_RES

<222> (15)

<223> AMIDATION, Glycine amide

<220>

<223> Synthetic Peptide

<400> 7

Met Thr Glu Trp Gly Glu Trp Asp Glu Ala Ser Ala Thr Ala Xaa
1               5                   10                  15

<210> 8

<211> 15

<212> PRT

<213> Artificial Sequence


<220>

<221> MOD_RES

<222> (15)

<223> AMIDATION,Glycine amide


<220>

<223> Synthetic Peptide


<400> 8

Leu Thr Glu Trp Ser Gln Trp Ser Glu Ala Asn Lys Ser Ala Xaa
1               5                   10                  15



<210> 9

<211> 15

<212> PRT

<213> Artificial Sequence


<220>

<221> MOD_RES

<222> (15)

<223> AMIDATION,Glycine amide

<220>

<223> Synthetic Peptide

<400> 9

Met Arg Pro Trp Thr Ala Trp Ser Glu Ala Thr Lys Leu Ala Xaa

1           5            10           15

<210> 10

<211> 15

<212> PRT

<213> Artificial Sequence

<220>

<221> MOD_RES

<222> (15)

<223> AMIDATION,Leucine amide

<220>

<223> Synthetic Peptide

<400> 10

Asp Lys Gly Lys Arg Met Arg Gln Arg Met Leu Lys Ala Gln Xaa

1           5            10           15

<210> 11

<211> 15

<212> PRT

<213> Artificial Sequence


<220>

<221> MOD_RES

<222> (15)

<223> AMIDATION,Glycine amide


<220>

<223> Synthetic Peptide


<400> 11

Met Lys Lys Arg His Arg Met Ile Lys Met Asn Pro Ala Asp Xaa
1               5                   10                  15


<210> 12

<211> 14

<212> PRT

<213> Artificial Sequence


<220>

<221> MOD_RES

<222> (14)

<223> AMIDATION,Glutamate amide

<220>

<223> Synthetic Peptide

<400> 12

Gly Lys Gly His Val Ile Arg Thr Arg Met Ile Gln Met Xaa

1                    5                    10

<210> 13

<211> 15

<212> PRT

<213> Artificial Sequence

<220>

<221> MOD_RES

<222> (15)

<223> AMIDATION,Lysine amide

<220>

<223> Synthetic Peptide

<400> 13

Pro Ala Pro Glu Thr Val Gln Arg Lys Lys Ala Arg Ile Arg Xaa

1                    5                    10                    15

<210> 14

<211> 15

<212> PRT

<213> Artificial Sequence


<220>

<221> MOD_RES

<222> (15)

<223> AMIDATION,Threonine amide


<220>

<223> Synthetic Peptide


<400> 14

Arg Tyr Met Thr Val Lys Lys Arg Phe Lys Ser Ser Gln Phe Xaa


**Description**

TECHNICAL FIELD

The present invention relates to peptides inhibiting vascular endothelial cell migration derived from a partial sequence of the protein of the amino acid sequence represented by any of SEQ ID NOs:1 to 3. Additionally, the present invention relates to agents for treating diseases related to pathological angiogenesis (abnormal angiogenesis), comprising the peptides.

BACKGROUND OF THE INVENTION

It has been known that pathological angiogenesis is deeply involved in the progress of diseases, for example, solid tumor, inflammatory diseases such as arthritis, ocular angiogenic diseases such as diabetic retinitism, and the like. The control of such pathological angiogenesis is effective for the treatment of these diseases *(Nature Medicine,* 1, 27-31 (1995); *New Engl. J. Med.,* 333, 1757-1763 (1995); *Cell,* 86, 353-364 (1996)). It is expected that angiogenesis-inhibiting agents capable of controlling pathological angiogenesis are also effective for the therapeutic treatment of psoralen, duodenum ulcer, infantile angioma, collateral angioplasty involved in ischemic cardiac diseases and the like *(Nature Medicine,* 1, 27-31 (1995)).

One step essential for angiogenesis is vascular endothelial cell migration. Thus, the inhibition of the vascular endothelial cell migration leads to the inhibition of angiogenesis *(Science,* 235, 442-447 (1987)).

DISCLOSURE OF THE INVENTION

An object of the present invention is to provide novel peptides which inhibit vascular endothelial cell migration and are useful as an agent for treating diseases related to pathological angiogenesis, for example, solid tumor, inflammatory diseases such as arthritis, ocular angiogenic diseases such as diabetic retinitis, or the like.

The present invention relates to peptides having an activity of inhibiting vascular endothelial cell migration represented by the following formula (I):

$$R^1\text{-}A\text{-}R^2 \tag{I}$$

wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a partial amino acid sequence of the amino acid sequence represented by any of SEQ ID NOs:1 to 3 wherein one or more amino acid residue may be substituted, deleted or added, or pharmaceutically acceptable salts thereof.

Additionally, the present invention provides peptides having an activity of inhibiting vascular endothelial cell migration, wherein A in formula (I) comprises an amino acid sequence of at least 5 consecutive residues, preferably 10 to 50 residues, more preferably 10 to 20 residues, in the amino acid sequence represented by any of SEQ ID NOs: 1 to 3, and wherein one or more amino acid residue may be substituted, deleted or added in the sequence, or pharmaceutically acceptable salts thereof.

Still additionally, the present invention provides peptides having an activity of inhibiting vascular endothelial cell migration, wherein A in formula (I) comprises an amino acid sequence of at least 5 consecutive residues, preferably 10 to 50 residues, more preferably 10 to.20 residues, in the amino acid sequence of the 400th to the 807th amino acids from the N terminus of the amino acid sequence represented by any of SEQ ID NOs:1 to 3, and wherein one or more amino acid residue may be substituted, deleted or added in the sequence, or pharmaceutically acceptable salts thereof.

Further, the present invention provides peptides having an activity of inhibiting vascular endothelial cell migration, wherein A in formula (I) is selected from the amino acid sequences represented by SEQ ID NOs:4 to 14, and wherein one or more amino acid residue may be deleted, substituted or added in the sequence, or pharmaceutically acceptable salts thereof.

Furthermore, the present invention provides peptides having an activity of inhibiting vascular endothelial cell migration, wherein A in formula (I) comprises an amino acid sequence represented by -Trp-$X^1$-$X^2$-Trp-$X^3$-, in which $X^1$ represents -Ser-, -Ile-, -Gly-, -Thr-, -Leu-, -Val- or -Ala-; $X^2$ represents -Glu-, -Thr-, -Pro-, -Gln-, -Ala-, -Asp-, -Ser-, -Asn- or -Gly-; and $X^3$ represents -Ser-, -Asp-, -Thr- or -Glu-, and wherein one or more amino acid residue may be deleted, substituted or added in the sequence, or pharmaceutically acceptable salts thereof.

Still furthermore, the present invention provides peptides having an activity of inhibiting vascular endothelial cell migration, wherein A in formula (I) comprises an amino acid sequence represented by -$X^4$-$X^5$-$X^6$-Trp-$X^1$-$X^2$-Trp-$X^3$-$X^7$-Cys-$X^8$-$X^9$-$X^{10}$-Cys-$X^{11}$-, in which $X^4$ represents -Met-, -Leu-, -Tyr-, -Thr-, -Val-, -Ile-, -Trp-, -Phe- or -Ser-; $X^5$ represents -Ser-, -Thr-, -Arg- or -Lys-; $X^6$ represents -Glu-, -Leu-, -Asn-, -Pro-, -Asp-, -Ile-, -Val-, -Gln- or -His-; $X^7$ represents -Glu-, -Pro-, -Asp-, -Ala-, -Gly- or -Ser-; $X^8$ represents -Ser-, -Asn-, -Thr-, -Gln- or -His-; $X^9$ represents -Lys-, -Ile-, -Val-, -Ser-Ser-, -Ala-, -Arg-, -Gln-, -Glu-, -Leu-, -Gly-, -Ser- or -Thr-Thr-; $X^{10}$ represents -Ser-, -Thr-, -Leu-, -Ile- or -Val-; $X^{11}$ represents -Gly-, -Asp-, -Glu- or -Ala-; and $X^1$, $X^2$ and $X^3$ each individually have the same meanings as described above, and wherein one or more amino acid residue may be deleted, substituted or added in the sequence, or pharmaceutically acceptable salts thereof.

Additionally, the present invention provides peptides having an activity of inhibiting vascular endothelial cell migration, wherein A in formula (I) comprises an amino acid sequence represented by -Cys-$X^8$-$X^9$-$X^{10}$-Cys-, in which $X^8$, $X^9$ and $X^{10}$ each individually have the same meanings as described above, and wherein one or more amino acid residue may be deleted, substituted or added in the sequence, or pharmaceutically acceptable salts thereof.

Additionally, the present invention provides peptides having an activity of inhibiting vascular endothelial cell migration, wherein A in formula (I) comprises an amino acid sequence represented by -$X^{12}$-$X^{13}$-$X^{14}$-$X^{15}$-, in which $X^{12}$, $X^{13}$ and $X^{15}$ are the same or different and represent -Lys-, -Arg-, -Gln- or -Glu-; and $X^{14}$ represents a single bond, -Met-, -His-, -Ala-, -Phe-, -Cys-, -Leu-, -Ile-, -Asn-, -Gln-, -Gly-, -Ser- or -Tyr-, and wherein one or more amino acid residue may be deleted, substituted or added in the sequence, or pharmaceutically acceptable salts thereof.

Still additionally, the present invention provides peptides having an activity of inhibiting vascular endothelial cell migration, wherein A in formula (I) comprises an amino acid sequence represented by -$X^{13}$-$X^{16}$-$X^{15}$-, in which $X^{16}$ represents a single bond, -Gly-, -Met-, -Gln-, -His-, -Thr-, -Ala-, -Ile-, -Lys-, -Phe-, -Cys-, -Leu-, -Asn-, -Ser-, -Val-, -Arg-, -Glu- or -Tyr-; and $X^{13}$ and $X^{15}$ each individually have the same meanings as described above, and wherein one or more amino acid residue may be deleted, substituted or added in the sequence, or pharmaceutically acceptable salts thereof.

Herein, the number of amino acid residues which may be substituted, deleted or added in each of the peptides or pharmaceutically acceptable salts thereof is preferably one to several tens of residues, more preferably one to several residues.

Furthermore, the present invention provides pharmaceutical compositions, agents for inhibiting vascular endothelial cell migration and agents for inhibiting abnormal angiogenesis, comprising each of the above peptides or a pharmaceutically acceptable salt thereof.

From another respect, the present invention relates to use of each of the above peptides or a pharmaceutically acceptable salt thereof for the manufacture of an agent for inhibiting vascular endothelial cell migration, use of each of the above peptides or a pharmaceutically acceptable salt thereof for the manufacture of an agent for inhibiting abnormal angiogenesis, a method for treating or preventing diseases related to vascular endothelial cell migration, comprising administering an effective amount of each of the above peptides or a pharmaceutically acceptable salt thereof, and a method for treating or preventing diseases related to abnormal angiogenesis, comprising administering an effective amount of each of the above peptides or a pharmaceutically acceptable salt thereof.

Hereinafter the compound represented by formula (I) is referred to as Compound (I).

In the definitions of each of the groups in formula (I), the alkanoyl includes linear or branched alkanoyls having 1 to 20 carbon atom(s), such as formyl, acetyl, propionyl, butyryl, isobutyryl, valeryl, isovaleryl, pivaloyl, hexanoyl, heptanoyl, lauroyl and eicosanoyl.

The aryl moieties in the aroyl and aryloxycarbonyl include aryls having 6 to 15 carbon atoms, such as phenyl and naphthyl.

The heteroaryl moieties in the heteroarylcarbonyl and heteroaryloxycarbonyl include furyl, thienyl, pyrrolyl, pyrazolyl, imidazolyl, oxazolyl, thiazolyl, pyridyl, pyridazinyl, pyrimidinyl, pyrazinyl, indolyl, indazolyl, benzimidazolyl, quinolyl, isoquinolyl, cinnolinyl, quinazolinyl, quinoxalinyl and naphthyridinyl.

The alkyl moieties in the alkoxycarbonyl and alkoxy include linear or branched alkyls having 1 to 20 carbon atom(s), such as methyl, ethyl, propyl, isopropyl, butyl, pentyl, hexyl, heptyl, decyl, dodecyl and eicosyl.

The substituents in the substituted alkanoyl, substituted alkoxycarbonyl and substituted alkoxy are the same or different and 1 to 3 substituent(s), such as hydroxy; carboxy; alicyclic alkyls having 3 to 8 carbon atoms, e.g., cyclopropyl, cyclobutyl, cyclopentyl, cyclohexyl, cycloheptyl, cyclooctyl, etc.; substituted or unsubstituted phenyl; or fluorenyl. The substituents in the substituted phenyl are the same or different and 1 to 3 substituent(s), such as alkyl, alkoxy, hydroxy, nitro, sulfo, cyano or halogen. The halogen includes each atom of fluorine, chlorine, bromine and iodine. The alkyl moieties in the alkyl and alkoxy as the substituent in the substituted phenyl have the same meaning as the alkyl moiety in the groups such as the above alkoxy.

The substituents in the substituted aroyl, substituted aryloxycarbonyl, substituted heteroarylcarbonyl and substituted heteroaryloxycarbonyl are the same or different and 1 to 3 substituent(s), and have the same meanings as the substituents for the above substituted phenyl.

The substituents in the substituted amino are the same or different and 1 to 2 substituent(s), such as substituted or unsubstituted alkyl, or substituted or unsubstituted aryl. The alkyl has the same meaning as the alkyl moiety in the groups such as the above alkoxy. The substituents in the substituted alkyl have the same meanings as the substituents in the groups such as the above substituted alkoxy. The aryl has the same meaning as the aryl moiety in the above aroyl or aryloxycarbonyl. The substituents in the substituted aryl have the same meanings as the substituents in the above aroyl or aryloxycarbonyl.

The amino acid sequence represented by SEQ ID NO:3 is known as a sequence derived from rats (*Cell,* 69, 95 (1992)), and the amino acid sequences of SEQ ID NOs:1 and 2 are amino acid sequences derived from humans and bovines, respectively.

Among Compounds (I), preferable compounds are compounds wherein A is selected from the amino acid sequences represented by SEQ ID NOs:4 to 14.

The expression "one or more amino acid residue may be substituted, deleted or added in the sequence" means that the number of single or plural amino acid residues substituted, deleted or added at optional and single or plural positions in the same sequence is one or more in total, the amino acid residues may be substituted, deleted or added simultaneously, and the amino acids to be substituted or added may be natural or non-natural. The natural amino acids include L-alanine, L-asparagine, L-aspartic acid, L-arginine, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine and L-cysteine.

The pharmaceutically acceptable salts of Compound (I) include acid addition salts, metal salts and organic base addition salts. The acid addition salts include inorganic salts such as a hydrochloride, a sulfate and a phosphate; and organic salts such as an acetate, a maleate, a fumarate, a tartrate and a citrate. The metal salts include alkali metal salts such as a sodium salt and a potassium salt; alkaline earth metal salts such as a magnesium salt and a calcium salt; an aluminium salt; and a zinc salt. The organic base addition salts include salts with a primary amine such as methylamine, ethylamine or aniline; a secondary amine such as dimethylamine, diethylamine pyrrolidine, piperidine, morpholine or piperazine; or a tertiary amine such as trimethylamine, triethylamine, N,N-dimethylaniline or pyridine; and ammonium salts.

The abbreviations for the amino acids and protective groups thereof for use in the specification are described below.

The abbreviations for the amino acids and the protective groups thereof are according to the recommendation of the IUPAC-IUB Joint Commission on Biochemical Nomenclature (*Eur. J. Biochem.,* 138, 9-37 (1984)) relating to biochemical nomenclature.

The following abbreviations mean the corresponding following amino acids, unless otherwise indicated.

| | |
|---|---|
| Ala: | L-alanine |
| Asn: | L-asparagine |
| Asp: | L-aspartic acid |
| Asx: | L-asparagine or L-aspartic acid |
| Arg: | L-arginine |
| Gln: | L-glutamine |
| Glu: | L-glutamic acid |
| Glx: | L-glutamine or L-glutamic acid |
| Gly: | glycine |
| His: | L-histidine |
| Ile: | L-isoleucine |
| Leu: | L-leucine |
| Lys: | L-lysine |
| Met: | L-methionine |
| Phe: | L-phenylalanine |
| Pro: | L-proline |
| Ser: | L-serine |
| Thr: | L-threonine |
| Trp: | L-tryptophan |
| Tyr: | L-tyrosine |
| Val: | L-valine |
| Cys: | L-cysteine |

The following abbreviations mean the corresponding following protective groups of the amino acids or side chain-protected amino acids.

Fmoc: 9-fluorenylmethyloxycarbonyl
Boc: t-butyloxycarbonyl
tBu: t-butyl
Trt: trityl
Pmc: 2,2,5,7,8-pentamethylchroman-6-sulfonyl

Fmoc-Asp(OtBu)-OH: $N^\alpha$-9-fluorenylmethyloxycarbonyl-L-aspartic acid-β-t-butyl ester
Fmoc-Glu(OtBu)-OH: $N^\alpha$-9-fluorenylmethyloxycarbonyl-L-glutamic acid-γ-t-butyl ester
Fmoc-Thr(tBu)-OH: $N^\alpha$-9-fluorenylmethyloxycarbonyl-O-t-butyl-L-threonine
Fmoc-Ser(tBu)-OH: $N^\alpha$ -9-fluorenylmethyloxycarbonyl-O-t-butyl-L-serine
Fmoc-Tyr(t-Bu)-OH: $N^\alpha$-9-fluorenylmethyloxycarbonyl-O-t-butyl-L-tyrosine
Fmoc-Arg(Pmc)-OH: $N^\alpha$-9-fluorenylmethyloxycarbonyl-$N^g$-2,2,5,7,8-pentamethylchroman-6-sulfonyl-L-arginine
Fmoc-Asn(Trt)-OH: $N^\alpha$-9-fluorenylmethyloxycarbonyl-$N^\beta$-trityl-L-asparagine
Fmoc-Gln(Trt)-OH: $N^\alpha$-9-fluorenylmethyloxycarbonyl-$N^\gamma$-trityl-L-glutamine
Fmoc-His(Trt)-OH: $N^\alpha$-9-fluorenylmethyloxycarbonyl-$N^{im}$-trityl-L-histidine
Fmoc-Lys(Boc)-OH: $N^\alpha$-9-fluorenylmethyloxycarbonyl-$N^\epsilon$-t-butyloxycarbonyl-L-lysine
Fmoc-Trp(Boc)-OH: $N^\alpha$-9-fluorenylmethyloxycarbonyl-$N^{in}$-t-butyloxycarbonyl-L-tryptophan

The following abbreviations mean the corresponding following reaction solvents, reaction reagents and the like.

PyBOP: benzotriazol-1-yloxytripyrrolidinophosphonium hexafluorophosphate
HOBt: N-hydroxybenzotriazole
DMF: N,N-dimethylformamide
TFA: trifluoroacetic acid
NMM: N-methylmorpholine
NMP: N-methylpyrrolidone

The method for producing Compound (I) is now described.

Compound (I) can be synthesized by general liquid-phase or solid-phase peptide synthetic methods, methods in appropriate combination thereof, or methods similar to these methods *(The Peptides, Analysis, Synthesis, Biology,* vol. 1, Erhard Gross and Johannes Meinhofer ed., Academic Press (1979); *ibid.,* vol. 2, Erhard Gross and Johannes Meinhofer ed., Academic Press (1980); *ibid.,* vol. 3, Erhard Gross and Johannes Meinhofer ed., Academic Press (1981); *Fundamentals and Experiments of Peptide Synthesis,* Nobuo Izumiya et *al.,* Maruzen (1985); *Development of Pharmaceutical Products (sequel),* vol. 14, Peptide Synthesis, supervised by Haruaki Yajima, Hirokawa Shoten (1991); and *International Journal of Peptide and Protein Research,* 35, 161 (1990)). Additionally, Compound (I) can also be synthesized with an automatic peptide synthesizer. Using appropriately side chain-protected $N^\alpha$-9-fluorenylmethyloxycarbonylamino acid or $N^\alpha$-t-butyloxycarbonylamino acid or the like, the synthesis of the peptide with a peptide synthesizer can be conducted with a commercially available peptide synthesizer, such as a peptide synthesizer manufactured by Shimadzu Corporation, a peptide synthesizer manufactured by Applied Biosystems Inc., U.S.A. (ABI), or a peptide synthesizer manufactured by Advanced ChemTech Inc., U.S.A. (ACT), following the synthesis program of each synthesizer.

The protected amino acids as the starting materials for the synthesis of Compound (I) and carrier resins are available from ABI, Shimadzu Corporation, Kokusan Chemicals Co., Ltd., Nova Biochem, Watanabe Chemicals Co., Ltd., ACT, the Peptide Research Institute, Co., Ltd. or the like.

Compound (I) thus obtained can be purified by high-performance liquid chromatography (hereinafter referred to as "HPLC"), using a reverse-phase silica gel column such as C-4, C-8, or C-18 type; column chromatography such as gel filtration, using partition resins, adsorption resins, ion exchange resins, silica gel, chemically modified silica gel, reverse-phase silica gel, alumina, diatomaceous earth, magnesium silicate or the like; thin-layer chromatography; or the like.

The pharmaceutically acceptable salts of Compound (I) can be obtained by ordinary methods. Specifically, the acid addition salts or organic base addition salts of Compound (I) can be obtained, for example, by dissolving Compound (I) in an aqueous solution of the corresponding acid or organic base and freeze-drying the resulting solution. Additionally, the metal salts of Compound (I) can be obtained by dissolving Compound (I) in an aqueous solution containing the corresponding metal ion and purifying the resulting solution by gel filtration or HPLC.

Specific examples of Compound (I) are now shown in Table 1.

Table 1

| Compound | Sequence |
|---|---|
| 1 | H-Met-Ser-Glu-Trp-Ile-Thr-Trp-Ser-Pro-Ala-Ser-Ile-Ser-Ala-Gly-NH₂ |
| 2 | H-Leu-Ser-Leu-Trp-Ser-Glu-Trp-Ser-Asp-Ala-Ser-Val-Thr-Ala-Gly-NH₂ |
| 3 | H-Tyr-Ser-Asn-Trp-Ser-Pro-Trp-Ser-Ala-Ala-Ser-Ser-Ser-Thr-Ala-Asp-NH₂ |
| 4 | H-Met-Thr-Glu-Trp-Gly-Glu-Trp-Asp-Glu-Ala-Ser-Ala-Thr-Ala-Gly-NH₂ |
| 5 | H-Leu-Thr-Glu-Trp-Ser-Gln-Trp-Ser-Glu-Ala-Asn-Lys-Ser-Ala-Gly-NH₂ |
| 6 | H-Met-Arg-Pro-Trp-Thr-Ala-Trp-Ser-Glu-Ala-Thr-Lys-Leu-Ala-Gly-NH₂ |
| 7 | H-Asp-Lys-Gly-Lys-Arg-Met-Arg-Gln-Arg-Met-Leu-Lys-Ala-Gln-Leu-NH₂ |
| 8 | H-Met-Lys-Lys-Arg-His-Arg-Met-Ile-Lys-Met-Asn-Pro-Ala-Asp-Gly-NH₂ |
| 9 | H-Gly-Lys-Gly-His-Val-Ile-Arg-Thr-Arg-Met-Ile-Gln-Met-Glu-NH₂ |
| 10 | H-Pro-Ala-Pro-Glu-Thr-Val-Gln-Arg-Lys-Lys-Ala-Arg-Ile-Arg-Lys-NH₂ |
| 11 | H-Arg-Tyr-Met-Thr-Val-Lys-Lys-Arg-Phe-Lys-Ser-Ser-Gln-Phe-Thr-NH₂ |

The physiological activities of the compound (I) are shown in Test Example.

Test Example 1

Measurement of activity inhibiting swine vascular endothelial cell migration:

Swine aorta endothelial cells (5th to 7th generations) were suspended in the 10% fetus calf serum-containing Dulbecco's modified Eagle medium and inoculated at a cell density of 32,000 cells/cm$^2$ in a 6-well plate, for culturing until the cells formed a single layer over the whole bottom of the culture dish. Subsequently, the culture broth was exchanged to the 0.1% bovine serum albumin-containing Dulbecco's modified Eagle medium containing a test compound at a concentration of 10 μmol/L, followed by culturing for 6 hours. Then, a part of the cells on the bottom of the culture dish was scraped off using a razor blade. Subsequently, the culture broth was exchanged to the 0.1% bovine serum albumin-containing Dulbecco's modified Eagle medium containing a test compound in a concentration of 10 μmol/L, followed by culturing for 24 hours. After immobilizing the cells, the number of the cells which had migrated on the bottom portion of the culture dish, where a part of the cells had been scraped off, was counted. The inhibition ratio of cell migration was calculated by the following equation.

Inhibition ratio of cell migration (%)

= (1 - the number of cells which had migrated in

the presence of the test compound/the number of

cells which had migrated in the absence of the test

compound) $\times$ 100

The results are shown in Table 2.

Table 2

| Compound | Inhibition ratio of cell migration (%) ± SD |
|---|---|
| 1 | 24.5±4.9 |
| 2 | 41.5±2.1 |
| 3 | 12.3±10.0 |
| 4 | 7.4±13.2 |
| 5 | 10.8±6.0 |
| 6 | 5.7±8.0 |
| 7 | 9.3±1.6 |
| 8 | 7.2±1.2 |
| 9 | 3.9±1.6 |
| 10 | 20.6±1.9 |
| 11 | 12.2±10.7 |
| SD : standard deviation | |

When a pharmaceutical composition, agent for inhibiting vascular endothelial cell migration or agent for inhibiting abnormal angiogenesis containing the peptide of the present invention or the pharmaceutically acceptable salt thereof is used, the agent can be administered in various forms, such as various injections, transcutaneous agents for mucosal administration, or oral agents, and can be used in various dosage forms corresponding to the above administration forms.

Examples of the dosage forms include injections such as intravenous injections, intramuscular injections, subcutaneous injections and intracutaneous injections; injections to be dissolved when used, such as suspension injections and freeze-dried preparations; preparations for transmucosal administration such as suppositories and transnasal preparations; and oral preparations such as tablets, capsules, granules and suspensions.

For the formulation of these dosage forms, generally known methods are applicable. For example, when the carrier for such dosage forms is a liquid, the peptide(s) of the present invention or pharmaceutically acceptable salt(s) thereof is dissolved or dispersed in the liquid. When the carrier for such dosage forms is a powder, the peptide(s) of the present invention or pharmaceutically acceptable salt(s) thereof is mixed with or adsorbed onto the powder. Furthermore, depending on the purpose, such dosage forms may contain pharmaceutically acceptable preservatives, stabilizers, antioxidants, excipients, binders, disintegrators, moisturizers, lubricants, colorants, flavors, corrigents, coatings, suspending agents, emulsifiers, dissolution auxiliary agents, buffers, isotonic agents, plasticizers, surfactants, soothing agents or the like.

The formulation carrier for use includes, for example, water, distilled water for injection, physiological saline, glucose, fructose, purified rice bran, lactose, starch, cellulose, methyl cellulose, carboxymethyl cellulose, hydroxypropyl cellulose, glycerin, mannitol, xylitol, sorbitol, glucuronic acid, hyaluronic acid, heparin, chitin, chitosan, glycine, alanine, proline, serine, cysteine, aspartic acid, glutamic acid, lysine, arginine, human serum albumin, human serum globulin, collagen, gelatin, alginic acid, talc, sodium citrate, calcium carbonate, calcium hydrogen phosphate, magnesium stearate, urea, silicone resin, sorbitan fatty acid ester, glycerin fatty acid ester, ascorbic acid, and $\alpha$-tocophenol.

The dose of the peptide of the present invention or a pharmaceutically acceptable salt thereof can vary, depending on the dosing method, the type of the peptide of the present invention or a pharmaceutically acceptable salt thereof, the age and symptoms of a patient and the like. Additionally, the dosing method can vary, depending on the symptoms and the dose. For example, the peptide of the present invention or a pharmaceutically acceptable salt thereof can be administered at a dose of 0.00001 to 100 mg/kg, preferably 0.0001 to 100 mg/kg, and more preferably 0.001 to 10 mg/kg, per day.

The present invention is now described in detail based on Examples.

BEST MODE FOR CARRYING OUT THE INVENTION

Example 1 Synthesis of Compound 1 (SEQ ID NO:4) (H-Met-Ser-Glu-Trp-Ile-Thr-Trp-Ser-Pro-Ala-Ser-Ile-Ser-Ala-Gly-NH$_2$) :

In the reactor of an automatic synthesizer manufactured by Shimadzu Corporation, 50 mg of a carrier resin (Rink Amide MBHA resin) bound with 23.5 µmol of a Fmoc-NH- group was placed for the following procedures according to the synthesis program of Shimadzu Corporation.

(a) The carrier resin was washed with 900 µl of DMF for 3 minutes, and the solution was discharged.
(b) After adding 900 µl of a 30% piperidine-DMF solution thereto, the resulting mixture was stirred for 4 minutes. The solution was discharged. The procedure was repeated once more.
(c) The carrier resin was washed with 900 µl of DMF for one minute, and the solution was discharged. The procedure was repeated 5 times.
    In such a manner, the resulting carrier resin bound with -NH$_2$ where the Fmoc group had been removed was obtained.
(d) Fmoc-Gly-OH (235 µmol), PyBOP (235 µmol), HOBt monohydrate (235 µmol) and NMM (352.5 µmol) were stirred in DMF (822.5 µl) for 3 minutes, and the resulting solution was added to the resin, followed by stirring for 60 minutes. Then, the solution was discharged.
(e) The carrier resin was washed with 900 µl of DMF for one minute. Then, the solution was discharged. The procedure was repeated 5 times.

In such a manner, the Fmoc-Gly-NH- group was synthesized on the carrier.
After subsequent washing and deprotection steps as described in (a) to (c), condensation reaction using Fmoc-Ala-OH instead of Fmoc-Gly-OH at the step (d) and the washing step (e), the Fmoc-Ala-Gly-NH- group was synthesized on the carrier. Using at the step (d), Fmoc-Ser(tBu)-OH, Fmoc-Ile-OH, Fmoc-Ser(tBu)-OH, Fmoc-Ala-OH, Fmoc-Pro-OH, Fmoc-Ser(tBu)-OH, Fmoc-Trp(Boc)-OH, Fmoc-Thr(tBu)-OH, Fmoc-Ile-OH, Fmoc-Trp(Boc)-OH, Fmoc-Glu(OtBu)-OH, Fmoc-Ser(tBu)-OH and Fmoc-Met-OH sequentially, the steps (a) to (e) were repeated to obtain the carrier resin bound with the protected peptides. After the additional washing and deprotection steps as described in (a) to (c), the carrier resin was washed with 900 µl of DMF for one minute, and the solution was discharged. The procedure was repeated 5 times. After sequential washing with methanol and butyl ether, the resulting carrier resin was dried under reduced pressure for 12 hours to obtain the carrier resin bound with the side chain-protected peptides. To the resulting carrier resin was added 400 µl of a mixture solution of TFA (90%) containing 5 mg/ml 2-methylindole, thioanisole (5%) and 1,2-ethanediol (5%), and the resulting mixture was allowed to stand at room temperature for 2 hours to remove the side chain-protecting groups and the peptide was cleaved from the resin. After the resin was separated by filtration, about 10 ml of ether was added to the obtained filtrate. Then, the resulting precipitate was recovered by centrifugation and decantation to obtain 42.7 mg of a crude peptide. The crude product was dissolved in 4 ml of DMSO and purified by HPLC using a reverse-phase column (YMC-Pack ODS-AM; 30 mm I.D. $\times$ 250 mm; manufactured by YMC). The product was eluted by a linear concentration gradient method adding an aqueous 90% acetonitrile solution containing 0.1% TFA to an aqueous 0.1% TFA solution, followed by detection at 220 nm to obtain a fraction containing Compound 1. The fraction was freeze-dried to obtain 13.7 mg of Compound 1.
Mass Spectrometry (FABMS); m/z = 1622.6 (M+H$^+$)
Amino acid analysis; Glx 1.0(1), Ser 4.0(4), Gly 1.1(1), Thr 0.9(1), Ala 2.1(2), Pro 1.0(1), Met 0.9(1), Ile 1.9(2), Trp was not analyzed.

Example 2 Synthesis of Compound 2 (SEQ ID NO:5) (H-Leu-Ser-Leu-Trp-Ser-Glu-Trp-Ser-Asp-Ala-Ser-Val-Thr-Ala-Gly-NH$_2$) :

In a manner similar to that in Example 1, using as a starting material 50 mg of a carrier resin (Rink Amide MBHA resin) bound with 23.5 µmol of a Fmoc-NH- group, Fmoc-Gly-OH, Fmoc-Ala-OH, Fmoc-Thr(tBu)-OH, Fmoc-Val-OH, Fmoc-Ser(tBu)-OH, Fmoc-Ala-OH, Fmoc-Asp(OtBu)-OH, Fmoc-Ser(tBu)-OH, Fmoc-Trp(Boc)-OH, Fmoc-Glu(OtBu)-OH, Fmoc-Ser(tBu)-OH, Fmoc-Trp(Boc)-OH, Fmoc-Leu-OH, Fmoc-Ser(tBu)-OH and Fmoc-Leu-OH were sequentially condensed thereto, followed by washing and drying to obtain a carrier resin bound with the side chain-protected peptides. In a manner similar to that in Example 1, then, the peptide was cleaved from the resin to obtain 38.3 mg of a crude peptide. In a manner similar to that in Example 1, further, purification using a reverse-phase column was carried out to obtain 9.4 mg of Compound 2.
Mass Spectrometry (FABMS); m/z = 1608.3 (M+H$^+$)
Amino acid analysis; Asx 1.0(1), Glx 0.9(1), Ser 3.9(4), Gly 1.1(1), Thr 1.0(1), Ala 2.1(2), Val 1.0(1), Leu 2.0(2), Trp was not analyzed.

Example 3 Synthesis of Compound 3 (SEQ ID NO:6) (H-Tyr-Ser-Asn-Trp-Ser-Pro-Trp-Ser-Ala-Ala-Ser-Ser-Ser-Thr-Ala-Asp-NH$_2$) :

In the reactor of an automatic synthesizer manufactured by ACT, 50 mg of a carrier resin (Rink Amide MBHA resin) bound with 27.5 µmol of a Fmoc-NH- group was placed. The following procedures were carried out according to the synthesis program of ACT.

(a) The carrier resin was washed with 1 ml of DMF for 3 minutes, and the solution was discharged.
(b) After adding 1 ml of a 25% piperidine-DMF solution thereto, the resulting mixture was stirred for 2 minutes. The solution was discharged. After additionally adding 1 ml of a 25% piperidine-DMF solution thereto, the resulting mixture was stirred for 10 minutes. The solution was discharged.
(c) The carrier resin was washed with 1 ml of DMF for one minute, and the solution was discharged. The procedure was repeated 6 times.
   In such a manner, the carrier resin bound with -NH$_2$ where the Fmoc group had been removed was obtained.
(d) After adding 300 µl of DMF, 275 µl of an NMP solution containing Fmoc-Asp(OtBu)-OH and HOBt monohydrate, individually at a concentration of 0.5 mol/L, 275 µl of a DMF solution containing PyBOP in a concentration of 0.5 mol/L and 138 µl of a DMF solution containing NMM in a concentration of 2.0 mol/L to the resin, the resulting resin mixture was stirred for 60 minutes, from which the solution was discharged.
(e) The carrier resin was washed with 1 ml of DMF, and then, the solution was discharged. The resin was washed under stirring with 1 ml of DMF for one minute, from which the solution was discharged. Furthermore, the resin was washed again with 1 ml of DMF, from which the solution was discharged.

In such a manner, the Fmoc-Asp(OtBu)-NH- group was synthesized on the carrier.
After subsequent washing and deprotection steps as described in (a) to (c), condensation reaction was carried out using a solution containing Fmoc-Ala-OH instead of Fmoc-Asp(OtBu)-OH at the step (d), followed by the washing step (e), to synthesize the Fmoc-Ala-Asp(OtBu)-NH- group on the carrier. In the following step (d), using Fmoc-Thr(tBu)-OH, Fmoc-Ser(tBu)-OH, Fmoc-Ser(tBu)-OH, Fmoc-Ser(tBu)-OH, Fmoc-Ala-OH, Fmoc-Ala-OH, Fmoc-Ser(tBu)-OH, Fmoc-Trp(Boc)-OH, Fmoc-Pro-OH, Fmoc-Ser(tBu)-OH, Fmoc-Trp(Boc)-OH, Fmoc-Asn(Trt)-OH, Fmoc-Ser(tBu)-OH and Fmoc-Tyr(tBu)-OH sequentially, the steps (a) to (e) were repeated to obtain the carrier resin bound with the protected peptides. After the additional washing and deprotection steps described in (a) to (c) were carried out, the carrier resin was washed with 900 µl of DMF for one minute, and then the solution was discharged. The procedure was repeated 5 times. After sequential washing with methanol and butyl ether, the resulting carrier resin was dried under reduced pressure for 12 hours to obtain the carrier resin bound with the side chain-protected peptides. To the resulting carrier resin was added 1 ml of a mixture solution of TFA (82.5%), thioanisole (5%), water (5%), ethyl methyl sulfide (3%), 1,2-ethanediol (2.5%), thiophenol (2%) and 2-methylindole (5 mg/ml), and the resulting mixture was allowed to stand at room temperature for 6 hours to remove the side chain-protecting groups and the peptide was cleaved from the resin. After the resin was separated by filtration, about 10 ml of ether was added to the obtained solution. The resulting precipitate was recovered by centrifugation and decantation to obtain 56.5 mg of a crude peptide. The crude product was dissolved in 1 ml of DMF, and the mixture was diluted up to 10 ml with 2 mol/L acetic acid and purified by HPLC using a reverse-phase column (CAPCELL PAK C18; 30 mm I.D. × 25 mm; manufactured by Shiseido Co., Ltd.) The product was eluted by a linear concentration gradient method adding an aqueous 90% acetonitrile solution containing 0.1% TFA to an aqueous 0.1% TFA solution, followed by detection at 220 nm, to obtain a fraction containing Compound 3. The fraction was freeze-dried to obtain 3.1 mg of Compound 3.
Mass Spectrometry (FABMS); m/z = 1716.1 (M+H$^+$)
Amino acid analysis; Asx 2.0(2), Ser 6.1(6), Thr 0.8(1), Ala 3.2(3), Pro 1.1(1), Tyr 0.9(1), Trp was not analyzed.

Example 4 Synthesis of Compound 4 (SEQ ID NO:7) (H-Met-Thr-Glu-Trp-Gly-Glu-Trp-Asp-Glu-Ala-Ser-Ala-Thr-Ala-Gly-NH$_2$) :

In a manner similar to that in Example 3, using a starting material 50 mg of a carrier resin (Rink Amide MBHA resin) bound with 27.5 µmol of a Fmoc-NH- group, Fmoc-Gly-OH, Fmoc-Ala-OH, Fmoc-Thr(tBu)-OH, Fmoc-Ala-OH, Fmoc-Ser(tBu)-OH, Fmoc-Ala-OH, Fmoc-Glu(OtBu)-OH, Fmoc-Asp(OtBu)-OH, Fmoc-Trp(Boc)-OH, Fmoc-Glu(OtBu)-OH, Fmoc-Gly-OH, Fmoc-Trp(Boc)-OH, Fmoc-Glu(OtBu)-OH, Fmoc-Thr(tBu)-OH and Fmoc-Met-OH were sequentially condensed thereto, followed by washing and drying to obtain the carrier resin bound with side chain-protected peptides. In a manner similar to that in Example 3, then, the peptide was cleaved from the resin to obtain 50.9 mg of a crude peptide. In a manner similar to that in Example 3, further, purification using a reverse-phase column was carried out to obtain 2.6 mg of Compound 4.

Mass Spectrometry (FABMS); m/z = 1640.0 (M+H$^+$)

Amino acid analysis; Asx 1.0(1), Glx 2.9(3), Ser 1.0(1), Gly 2.1(2), Thr 2.0(2), Ala 3.1(3), Met 0.9(1), Trp was not analyzed.

Example 5 Synthesis of Compound 5 (SEQ ID NO:8) (H-Leu-Thr-Glu-Trp-Ser-Gln-Trp-Ser-Glu-Ala-Asn-Lys-Ser-Ala-Gly-NH$_2$) :

In a manner similar to that in Example 3, using as a starting material 50 mg of a carrier resin (Rink Amide MBHA resin) bound with 27.5 µmol of a Fmoc-NH- group, Fmoc-Gly-OH, Fmoc-Ala-OH, Fmoc-Ser(tBu)-OH, Fmoc-Lys(Boc)-OH, Fmoc-Asn(Trt)-OH, Fmoc-Ala-OH, Fmoc-Glu(OtBu)-OH, Fmoc-Ser(tBu)-OH, Fmoc-Trp(Boc)-OH, Fmoc-Gln(Trt)-OH, Fmoc-Ser(tBu)-OH, Fmoc-Trp(Boc)-OH, Fmoc-Glu(OtBu)-OH, Fmoc-Thr(tBu)-OH and Fmoc-Leu-OH were sequentially condensed thereto, followed by washing and drying to obtain the carrier resin bound with side chain-protected peptides. In a manner similar to that in Example 3, then, the peptide was cleaved from the resin to obtain 16.4 mg of a crude peptide. In a manner similar to that in Example 3, further, purification using a reverse-phase column was carried out to obtain 4.2 mg of Compound 5.

Mass Spectrometry (FABMS); m/z = 1692.8 (M+H$^+$)

Amino acid analysis; Asx 0.9(1), Glx 3.0(3), Ser 2.9(3), Gly 1.1(1), Thr 1.0(1), Ala 2.0(2), Leu 1.1(1), Lys 1.0(1), Trp was not analyzed.

Example 6 Synthesis of Compound 6 (SEQ ID NO:9) (H-Met-Arg-Pro-Trp-Thr-Ala-Trp-Ser-Glu-Ala-Thr-Lys-Leu-Ala-Gly-NH$_2$) :

In a manner similar to that in Example 3, using as a starting material 50 mg of a carrier resin (Rink Amide MBHA resin) bound with 27.5 µmol of Fmoc-NH- group, Fmoc-Gly-OH, Fmoc-Ala-OH, Fmoc-Leu-OH, Fmoc-Lys(Boc)-OH, Fmoc-Thr(tBu)-OH, Fmoc-Ala-OH, Fmoc-Glu(OtBu)-OH, Fmoc-Ser(tBu)-OH, Fmoc-Trp(Boc)-OH, Fmoc-Ala-OH, Fmoc-Thr(tBu)-OH, Fmoc-Trp(Boc)-OH, Fmoc-Pro-OH, Fmoc-Arg(Pmc)-OH and Fmoc-Met-OH was sequentially condensed thereto, followed by washing and drying to obtain the carrier resin bound with side chain-protected peptides. In a manner similar to that in Example 3, then, the peptide was cleaved from the resin to obtain 80.1 mg of a crude peptide. In a manner similar to that in Example 3, further, purification using a reverse-phase column was carried out to obtain 3.9 mg of Compound 6.

Mass Spectrometry (FABMS); m/z = 1704.9 (M+H$^+$)

Amino acid analysis; Glx 1.0(1), Ser 1.0(1), Gly 1.2(1), Arg 0.6(1), Thr 2.1(2), Ala 3.2(3), Pro 1.0(1), Met 0.7(1), Leu 1.1(1), Lys 1.0(1), Trp was not analyzed.

Example 7 Synthesis of Compound 7 (SEQ ID NO:10) (H-Asp-Lys-Gly-Lys-Arg-Met-Arg-Gln-Arg-Met-Leu-Lys-Ala-Gln-Leu-NH$_2$) :

In a manner similar to that in Example 3, using as a starting material 50 mg of a carrier resin (Rink Amide MBHA resin) bound with 27.5 µmol of a Fmoc-NH- group, Fmoc-Leu-OH, Fmoc-Gln(Trt)-OH, Fmoc-Ala-OH, Fmoc-Lys(Boc)-OH, Fmoc-Leu-OH, Fmoc-Met-OH, Fmoc-Arg(Pmc)-OH, Fmoc-Gln(Trt)-OH, Fmoc-Arg(Pmc)-OH, Fmoc-Met-OH, Fmoc-Arg(Pmc)-OH, Fmoc-Lys(Boc)-OH, Fmoc-Gly-OH, Fmoc-Lys(Boc)-OH and Fmoc-Asp(OtBu)-OH were sequentially condensed thereto, followed by washing and drying to obtain the carrier resin bound with side chain-protected peptides. To the resulting carrier was added 1 ml of a mixture solution of TFA (82.5%), thioanisole (5%), water (5%), ethyl methyl sulfide (3%), 1,2-ethanedithiol (2.5%) and thiophenol (2%), and the resulting solution was allowed to stand at room temperature for 8 hours to remove the side chain-protecting groups and the peptide was cleaved from the resin to obtain 82.6 mg of a crude peptide. In a manner similar to that in Example 3, then, purification by HPLC using a reverse-phase column was carried out to obtain 10.0 mg of Compound 7.

Mass Spectrometry (FABMS); m/z = 1857.8 (M+H$^+$)

Amino acid analysis; Asx 0.9(1), Glx 2.1(2), Gly 1.0(1), Arg 2.9(3), Ala 1.1(1), Met 2.0(2), Leu 2.1(2), Lys 3.0(3).

Example 8 Synthesis of Compound 8 (SEQ ID NO:11) (H-Met-Lys-Lys-Arg-His-Arg-Met-Ile-Lys-Met-Asn-Pro-Ala-Asp-Gly-NH$_2$) :

In a manner similar to that in Example 3, using as a starting material 50 mg of a carrier resin (Rink Amide MBHA resin) bound with 27.5 µmol of a Fmoc-NH- group, Fmoc-Gly-OH, Fmoc-Asp(tBu)-OH, Fmoc-Ala-OH, Fmoc-Pro-OH, Fmoc-Asn(Trt)-OH, Fmoc-Met-OH, Fmoc-Lys(Boc)-OH, Fmoc-Ile-OH, Fmoc-Met-OH, Fmoc-Arg(Pmc)-OH, Fmoc-His(Trt)-OH, Fmoc-Arg(Pmc)-OH, Fmoc-Lys(Boc)-OH, Fmoc-Lys(Boc)-OH and Fmoc-Met-OH were sequentially condensed thereto, followed by washing and drying to obtain the carrier resin bound with side chain-protected peptides. In a manner similar to that in Example 7, then, the peptide was cleaved from the resin to obtain 114.3 mg of a crude peptide. In a manner similar to that in Example 3, further, purification using a reverse-phase column was carried out to obtain 26.7 mg of Compound 8.

Mass Spectrometry (FABMS); m/z = 1812.6 (M+H$^+$)

Amino acid analysis; Asx 2.0(2), Gly 1.1(1), His 1.0(1), Arg 2.0(2), Ala 1.0(1), Pro 1.1(1), Met 2.8(3), Ile 1.0(1), Lys 3.0(3).

Example 9 Synthesis of Compound 9 (SEQ ID NO:12) (H-Gly-Lys-Gly-His-Val-Ile-Arg-Thr-Arg-Met-Ile-Gln-Met-Glu-NH$_2$) :

In a manner similar to that in Example 3, using as a starting material 50 mg of a carrier resin (Rink Amide MBHA resin) bound with 27.5 µmol of Fmoc-NH- group, Fmoc-Glu(OtBu)-OH, Fmoc-Met-OH, Fmoc-Gln(Trt)-OH, Fmoc-Ile-OH, Fmoc-Met-OH, Fmoc-Arg(Pmc)-OH, Fmoc-Thr(tBu)-OH, Fmoc-Arg(Pmc)-OH, Fmoc-Ile-OH, Fmoc-Val-OH, Fmoc-His(Trt)-OH, Fmoc-Gly-OH, Fmoc-Lys(Boc)-OH and Fmoc-Gly-OH were sequentially condensed thereto, followed by washing and drying to obtain the carrier resin bound with side chain-protected peptides. In a manner similar to that in Example 7, then, the peptide was cleaved from the resin to obtain 66.6 mg of a crude peptide. In a manner similar to that in Example 3, further, purification using a reverse-phase column was carried out to obtain 17.6 mg of Compound 9.

Mass Spectrometry (FABMS); m/z = 1654.6 (M+H$^+$)

Amino acid analysis; Glx 2.0(2), Gly 2.2(2), His 1.0(1), Arg 2.2(2), Thr 1.1(1), Val 0.6(1), Met 2.2(2), Ile 1.7(2), Lys 1.0(1).

Example 10 Synthesis of Compound 10 (SEQ ID NO:13) (H-Pro-Ala-Pro-Glu-Thr-Val-Gln-Arg-Lys-Lys-Ala-Arg-Ile-Arg-Lys-NH$_2$):

In a manner similar to that in Example 3, using as a starting material 50 mg of a carrier resin (Rink Amide MBHA resin) bound with 27.5 µmol of a Fmoc-NH- group, Fmoc-Lys(Boc)-OH, Fmoc-Arg(Pmc)-OH, Fmoc-Ile-OH, Fmoc-Arg(Pmc)-OH, Fmoc-Ala-OH, Fmoc-Lys(Boc)-OH, Fmoc-Lys(Boc)-OH, Fmoc-Arg(Pmc)-OH, Fmoc-Gln(Trt)-OH, Fmoc-Val-OH, Fmoc-Thr(tBu)-OH, Fmoc-Glu(OtBu)-OH, Fmoc-Pro-OH, Fmoc-Ala-OH and Fmoc-Pro-OH were sequentially condensed thereto, followed by washing and drying to obtain the carrier resin bound with side chain-protected peptides. In a manner similar to that in Example 7, then, the peptide was cleaved from the resin to obtain 85.9 mg of a crude peptide. In a manner similar to that in Example 3, further, purification using a reverse-phase column was carried out to obtain 23.7 mg of Compound 10.

Mass Spectrometry (FABMS); m/z = 1776.7 (M+H$^+$)

Amino acid analysis; Glx 2.0(2), Arg 3.0(3), Thr 1.0(1), Ala 2.1(2), Pro 1.8(2), Val 1.0(1), Ile 1.0(1), Lys 3.1(3).

Example 11 Synthesis of Compound 11 (SEQ ID NO:14) (H-Arg-Tyr-Met-Thr-Val-Lys-Lys-Arg-Phe-Lys-Ser-Ser-Gln-Phe-Thr-NH$_2$) :

In a manner similar to that in Example 1, using as a starting material 50 mg of a carrier resin (Rink Amide MBHA resin) bound with 27.5 µmol of a Fmoc-NH- group, Fmoc-Thr(tBu)-OH, Fmoc-Phe-OH, Fmoc-Gln(Trt)-OH, Fmoc-Ser(tBu)-OH, Fmoc-Ser(tBu)-OH, Fmoc-Lys(Boc)-OH, Fmoc-Phe-OH, Fmoc-Arg(Pmc)-OH, Fmoc-Lys(Boc)-OH, Fmoc-Lys(Boc)-OH, Fmoc-Val-OH, Fmoc-Thr(tBu)-OH, Fmoc-Met-OH, Fmoc-Tyr(tBu)-OH and Fmoc-Arg(Pmc)-OH were sequentially condensed thereto, followed by washing and drying to obtain the carrier resin bound with side chain-protected peptides. In a manner similar to that in Example 7, then, the peptide was cleaved from the resin to obtain 61.5 mg of a crude peptide. In the same manner similar to that in Example 3, further, purification using a reverse-phase column was carried out to obtain 2.5 mg of Compound 11.

Mass Spectrometry (FABMS); m/z = 1906.1 (M+H$^+$)

Amino acid analysis; Glx 1.1(1), Ser 2.1(2), Arg 1.9(2), Thr 2.0(2), Tyr 1.0(1), Val 0.9(1), Met 1.0(1), Phe 2.1(2), Lys 3.0(3).

INDUSTRIAL APPLICABILITY

The present invention provides novel peptides inhibiting vascular endothelial cell migration, which are useful as an agent for treating diseases related to pathological angiogenesis, for example, solid tumor, inflammatory diseases such as arthritis, ocular angiogenic diseases such as diabetic retinitis, or the like.

Sequence Listing Free Text

SEQ ID NO:4

    Artificial sequence: synthetic peptide
    Amidation: glycine amide

SEQ ID NO:5

    Artificial sequence: synthetic peptide
    Amidation: glycine amide

SEQ ID NO:6

    Artificial sequence: synthetic peptide
    Amidation: aspartic acid amide

SEQ ID NO:7

    Artificial sequence: synthetic peptide
    Amidation: glycine amide

SEQ ID NO:8

    Artificial sequence: synthetic peptide
    Amidation: glycine amide

SEQ ID NO:9

    Artificial sequence: synthetic peptide
    Amidation: glycine amide

SEQ ID NO:10

    Artificial sequence: synthetic peptide
    Amidation: leucine amide

SEQ ID NO:11

    Artificial sequence: synthetic peptide
    Amidation: glycine amide

SEQ ID NO:12

    Artificial sequence: synthetic peptide
    Amidation:glutamic acid amide

SEQ ID NO:13

    Artificial sequence: synthetic peptide
    Amidation: lysine amide

SEQ ID NO:14

    Artificial sequence: synthetic peptide
    Amidation: threonine amide

**Claims**

1. A peptide having an activity of inhibiting vascular endothelial cell migration represented by the following formula (I):

$$R^1\text{-A-}R^2 \tag{I}$$

wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ repressents hydroxy, substituted or unsubstituted alkoxy, or substituted or unsubstituted amino; and A represents a partial amino acid seauence of the amino acid sequences represented by any of SEQ ID NOs:1 to 3 wherein one or more amino acid residue may be substituted, deleted or added, or a pharmaceutically acceptable salt thereof.

2. The peptide according to claim 1, wherein A comprises an amino acid sequence of at least 5 consecutive residues in the amino acid sequence represented by any of SEQ ID NOs:1 to 3, and wherein one or more amino acid residue may be substituted, deleted or added in the sequence, or a pharmaceutically acceptable salt thereof.

3. The peptide according to claim 1, wherein A comprises an amino acid sequence of at least 5 consecutive residues in the amino acid sequence of the 400th to the 807th amino acids from the N terminus of the amino acid sequence represented by any of SEQ ID NOs:1 to 3, and wherein one or more amino acid residue may be substituted, deleted or added in the sequence, or a pharmaceutically acceptable salt thereof.

4. The peptide according to claim 1, wherein A is selected from the amino acid sequences represented by SEQ ID NOs:4 to 14, and wherein one or more amino acid residue may be deleted, substituted or added in the sequence, or a pharmaceutically acceptable salt thereof.

5. The peptide according to claim 1, wherein A comprises an amino acid sequence represented by -Trp-$X^1$-$X^2$-Trp-$X^3$-, in which $X^1$ represents -Ser-, -Ile-, -Gly-, -Thr-, -Leu-, -Val- or -Ala-; $X^2$ represents -Glu-, -Thr-, -Pro-, -Gln-, -Ala-, -Asp-, -Ser-, -Asn- or -Gly-; and $X^3$ represents -Ser-, -Asp-, -Thr- or -Glu-, and wherein one or more amino acid residue in may be deleted, substituted or added in the sequence, or a pharmaceutically acceptable salt thereof.

6. The peptide according to claim 1, wherein A comprises an amino acid sequence represented by -$X^4$-$X^5$-$X^6$-Trp-$X^1$-$X^2$-Trp-$X^3$-$X^7$-Cys-$X^8$-$X^9$-$X^{10}$-Cys-$X^{11}$-, in which $X^1$ represents -Ser-, -Ile-, -Gly-, -Thr-, -Leu-, -Val- or -Ala-; $X^2$ represents -Glu-, -Thr-, -Pro-, -Gln-, -Ala-, -Asp-, -Ser-, -Asn- or -Gly-; $X^3$ represents -Ser-, -Asp-, -Thr- or -Glu-; $X^4$ represents- -Met-, -Leu-, -Tyr-, -Thr-, -Val-, -Ile-, -Trp-, -Phe- or -Ser-; $X^5$ represents -Ser-, -Thr-, -Arg- or -Lys-; $X^6$ represents -Glu-, -Leu-, -Asn-, -Pro-, -Asp-, -Ile-, -Val-, -Gln- or -His-; $X^7$ represents -Glu-, -Pro-, -Asp-, -Ala-, -Gly- or -Ser-; $X^8$ represents -Ser-, -Asn-, -Thr-, -Gln- or -His-; $X^9$ represents -Lys-, -Ile-, -Val-, -Ser-Ser-, -Ala-, -Arg-, -Gln-, -Glu-, -Leu-, -Gly-, -Ser- or -Thr-Thr-; $X^{10}$ represents -Ser-, -Thr-, -Leu-, -Ile- or -Val-; and $X^{11}$ represents -Gly-, -Asp-, -Glu- or -Ala-, and wherein one or more amino acid residue may be deleted, substituted or added in the sequence, or a pharmaceutically acceptable salt thereof.

7. The peptide according to claim 1, wherein A comprises an amino acid sequence represented by -Cys-$X^8$-$X^9$-$X^{10}$-Cys-, in which $X^8$ represents -Ser-, -Asn-, -Thr-, -Gln- or -His-; $X^9$ represents -Lys-, -Ile-, -Val-, -Ser-Ser-, -Ala-, -Arg-, -Gln-, -Glu-, -Leu-, -Gly-, -Ser- or -Thr-Thr-; $X^{10}$ represents -Ser-, -Thr-, -Leu-, -Ile- or -Val-, and wherein one or more amino acid residue may be deleted, substituted or added in the sequence, or a pharmaceutically acceptable salt thereof.

8. The peptide according to claim 1, wherein A comprises an amino acid sequence represented by -$X^{12}$-$X^{13}$-$X^{14}$-$X^{15}$-, in which $X^{12}$, $X^{13}$ and $X^{15}$ are the same or different and represent -Lys-, -Arg-, -Gln- or -Glu-; and $X^{14}$ represents a single bond, -Met-, -His-, -Ala-, -Phe-, -Cys-, -Leu-, -Ile-, -Asn-, -Gln-, -Gly-, -Ser- or -Tyr-, ana wherein one or more amino acid residue may be deleted, substituted or added in the sequence, or a pharmaceutically acceptable salt thereof.

9. The peptide according to claim 1, wherein A comprises an amino acid sequence represented by -$X^{13}$-$X^{16}$-$X^{15}$-, in which $X^{13}$ and $X^{15}$ are the same or different and represent -Lys-, -Arg-, -Gin- or -Glu-; $X^{16}$ represents a single bond, -Gly-, -Met-, -Gln-, -His-, -Thr-, -Ala-, -Ile-, -Lys-, -Phe-, -Cys-, -Leu-, -Asn-, -Ser-, -Val-, -Arg-, -Glu- or -Tyr-, and wherein one or more amino acid residue may be deleted, substituted or added in the sequence, or a pharmaceutically acceptable salt thereof.

10. A pharmaceutical composition, comprising the peptide according to any one of claims 1 to 9, or a pharmaceutically acceptable salt thereof.

11. Use of the peptide according to any one of claims 1 to 9 or a pharmaceutically acceptable salt thereof for the manufacture of an agent for inhibiting vascular endothelial cell migration, or for treating or preventing a disease related to vascular endothelial cell migration.

12. Use of the peptide according to any one of claims 1 to 9 or a pharmaceutically acceptable salt thereof for the manufacture of an agent for inhibiting abnormal angiogenesis or for treating or preventing disease related to abnormal angiogenesis.

| INTERNATIONAL SEARCH REPORT | International application No. |
|---|---|
| | PCT/JP00/00703 |

**A. CLASSIFICATION OF SUBJECT MATTER**
Int.Cl$^7$   C07K 4/00, C07K 14/47, A61K 38/02, A61K 38/16

According to International Patent Classification (IPC) or to both national classification and IPC

**B. FIELDS SEARCHED**

Minimum documentation searched (classification system followed by classification symbols)
Int.Cl$^7$   C07K 4/00, C07K 14/47, A61K 38/02, A61K 38/16

Documentation searched other than minimum documentation to the extent that such documents are included in the fields searched

Electronic data base consulted during the international search (name of data base and, where practicable, search terms used)
CA(STN), REGISTRY(STN), SwissProt/PIR/GeneSeq

**C. DOCUMENTS CONSIDERED TO BE RELEVANT**

| Category* | Citation of document, with indication, where appropriate, of the relevant passages | Relevant to claim No. |
|---|---|---|
| X | Avlhu Klar et al. "F-Spondin: A Gene Expressed at High Levels in the Floor Plate Encodes a Secreted Protein That Promotes Neural Cell Adhesion and Neurite Extension", Cell, Vol.69, No.1, p.95-110(1992) | 1-14 |
| X | WO, 93/20196, A (UNIV COLUMBIA NEW YORK), 14 October, 1993 (14.10.93), & EP, 670895, A  & JP, 7-508402, A & US, 5750502, A | 1-14 |
| PX | EP, 905235, A2 (KYOWA HAKKO KOGYO KK.), 31 March, 1999 (31.03.99) & JP, 11-123092, A  & AU, 7993598, A | 1-14 |

☐ Further documents are listed in the continuation of Box C.   ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 28 April, 2000 (28.04.00) | 16 May, 2000 (16.05.00) |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

International application No.

PCT/JP00/00703

| Box I | Observations where certain claims were found unsearchable (Continuation of item 1 of first sheet) |
| --- | --- |

This international search report has not been established in respect of certain claims under Article 17(2)(a) for the following reasons:

1. ☒ Claims Nos.: 15-16
   because they relate to subject matter not required to be searched by this Authority, namely:

   The subject matter of claims 15-16 corresponds to a method for treatment of the human body.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

| Box II | Observations where unity of invention is lacking (Continuation of item 2 of first sheet) |
| --- | --- |

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**   ☐   The additional search fees were accompanied by the applicant's protest.

   ☐   No protest accompanied the payment of additional search fees.

Form PCT/ISA/210 (continuation of first sheet (1)) (July 1992)